**Europäisches Patentamt**

**European Patent Office**

**Office européen des brevets**

(11) Numéro de publication: **0 243 259**
**A2**

# (12) DEMANDE DE BREVET EUROPEEN

(21) Numéro de dépôt: **87400910.3**

(22) Date de dépôt: **17.04.87**

(51) Int. Cl.³: **A 61 M 16/01**
**A 61 M 16/18**

(30) Priorité: **22.04.86 FR 8605989**

(43) Date de publication de la demande:
**28.10.87 Bulletin 87/44**

(84) Etats contractants désignés:
**AT BE CH DE ES GB GR IT LI LU NL SE**

(71) Demandeur: **Corfa, Yves-Paul**
**La Briantière Oudon**
**F-44150 Ancenis(FR)**

(72) Inventeur: **Corfa, Yves-Paul**
**La Briantière Oudon**
**F-44150 Ancenis(FR)**

(74) Mandataire: **Lemonnier, André**
**4 Boulevard Saint-Denis**
**F-75010 Paris(FR)**

(54) **Doseur-évaporateur de liquide dans un circuit de gaz.**

(57) La présente invention concerne un doseur-évaporateur de liquide dans un circuit de gaz qui combine un dispositif de mesure du débit gazeux, une pompe doseuse de volumes du liquide et une chambre de vaporisation du liquide débité dans le gaz.

Conformément à l'invention la pompe doseuse (13–20) fonctionne selon un cycle asservi à des impulsions émises par le dispositif de mesure du débit gazeux, chaque impulsion correspondant à un volume gazeux débité fixe et constant, la pompe doseuse (13–20) de course réglable (28–30) effectuant un cycle par impulsion reçue du dispositif de mesure et la chambre de vaporisation étant consituée par une enceinte (26) traversée par le courant gazeux dans laquelle les volumes de liquide dosés sont pulvérisés (25), ladite enceinte étant séparée de la sortie des gaz par une paroi poreuse (27).

L'invention est applicable notamment aux installations pour l'anesthésie.

Fig.1

EP 0 243 259 A2

"Doseur-évaporateur de liquide dans un circuit de gaz"

La présente invention est relative à un doseur-évaporateur de liquide dans un circuit de gaz destiné en particulier aux installations médicales pour l'anesthésie.

Dans toutes les installations d'anesthésie destinées à alimenter en gaz respirable un patient en cours d'opération est inclus un évaporateur. Le rôle de cet évaporateur est d'ajouter au gaz une quantité définie de vapeur d'un anesthésique volatil ou de tout autre liquide.

Dans la très grande généralité des cas, le dosage s'obtient en dérivant une partie réglable des gaz et en faisant balayer par cette partie dérivée la surface d'un bac contenant l'anesthésique en phase liquide. Le dosage dépend alors de la vitesse de passage des gaz et de la température, ce qui le rend doublement imprécis. D'autre part le remplissage du bac oblige à des manipulations d'un liquide qui est un anesthésique et un solvant extrêmement puissant. Ces appareils sont munis d'une abaque de correction pour la température du local, parfois pour le débit de gaz, et sont généralement en laiton massif pour éviter que le refroidissement dû à l'évaporation ne soit trop rapidement ressenti.

GB-A-1.243.765 décrit un respirateur médical comportant un injecteur de liquide pour injecter une quantité de liquide dans une quantité prédéterminée de gaz dans lequel le gaz est, pendant la période d'expiration d'un cycle respiratoire, introduit sous pression dans une enceinte de volume connu, l'injecteur de liquide comportant une pompe à liquide dont le volume est contrôlé par la pression du gaz dans l'enceinte et la pompe injectant le volume de liquide ainsi dosé dans le gaz pendant la période d'inspiration sous une pression qui décroît avec la pression du gaz dans l'enceinte. La quantité de liquide injectée dans les volumes unitaires constituant le volume global inspiré varie donc lorsque la pression du gaz dans l'enceinte varie pendant la période d'inspiration. Or il est techniquement nécessaire que la quantité de liquide soit proportionnelle au volume de gaz et non à sa pression. Ce document ne suggère pas de procéder à un mélange par pulvérisation du liquide dans le gaz avant de vaporiser complètement le liquide.

DE-A-3.522.452 décrit un appareil pour vaporiser un ou des liqui-

3

des dans un courant gazeux dans lequel un micro-ordinateur reçoit en entrée ou contrôle le débit de gaz admis dans la chambre de vaporisation, les soupapes d'admission dans ladite chambre des liquides à vaporiser alimentées par des réservoirs sous pression, l'énergie calorifique assurant la vaporisation et la pression dans l'enceinte du mélange respiratoire pour vérifier une équation entre ces différents facteurs. Le contrôle s'effectue sur l'énergie calorifique fournie à la chambre de vaporisation pour vaporiser les liquides en obtenant une pression donnée. Les soupapes de dosage des liquides qui peuvent être commandées manuellement, ne dosent pas en volume et ne sont pas asservies au débit gazeux.

Ces deux brevets, quoique concernant la préparation d'un gaz respiratoire contenant un ou des liquides anesthésiants ou humidificateurs, n'envisagent pas et ne suggèrent pas de doser la quantité en fonction du débit de gaz respiratoire. En outre le dosage du volume injecté est complexe. Dans GB-A- 1.243.765 la dose de liquide injectée est fonction de la pression du gaz et du réglage du ressort de contre-pression. Dans DE-A-3.522.452 le réglage est incorporé dans le programme du micro-ordinateur.

L'appareil objet de la présente invention est destiné à assurer un dosage rigoureux indépendant de tous les autres facteurs. Il ne demande pas de manipulations de liquide. Il fonctionne en combinaison avec un mélangeur tel que ceux décrits dans FR-A-2.548.908 et son certificat d'addition FR-A-2.561.927 qui émettent des impulsions caractéristiques du débit ou de tout autre équipement de mesure de débit du gaz capable de fournir un signal à chaque passage d'un volume défini du gaz véhiculant le liquide vaporisé.

La présente invention a en conséquence pour objet un doseur-évaporateur de liquide dans un circuit de gaz qui combine un dispositif de mesure du débit gazeux, une pompe doseuse de volumes du liquide et une chambre de vaporisation du liquide débité dans le gaz caractérisé en ce que la pompe doseuse fonctionne selon un cycle asservi à des impulsions émises par le dispositif de mesure du débit gazeux, chaque impulsion correspondant à un volume gazeux débité fixe et constant, la pompe doseuse de course réglable effectuant un cycle par impulsion reçue du dispositif de mesure et la chambre de vaporisation étant constituée par une enceinte traversée par le courant gazeux dans laquelle les volumes de liquide dosés sont pulvérisés, ladite enceinte étant séparée de la sortie des gaz par une paroi poreuse. La paroi poreuse est en général tubulaire et elle a pour but d'empêcher l'entraînement par les gaz de gouttelettes.

Selon une autre caractéristique la paroi poreuse présente une capacité et une conductibilité thermiques importantes pour réguler et uniformiser la température des gaz en sortie. Selon un mode de réalisation la paroi poreuse est réalisée en poudre magnétique frittée et elle est entourée par un enroulement électrique parcouru par un courant alternatif basse tension qui est régulé pour assurer la température en sortie voulue du mélange gazeux anesthésique. Selon un autre mode de réalisation la paroi poreuse est réa-

0243259

lisée en un matériau amagnétique et elle est chauffée par une bobine d'induction haute fréquence placée à l'intérieur de ladite paroi poreuse tubulaire, ceci dans le but d'éviter les pertes diélectriques.

Selon une autre caractéristique, qui s'applique notamment au cas d'un liquide non volatil tel que l'eau, l'évaporation est assurée, avant le passage à travers la paroi poreuse, par un élément formant résistance électrique sur lequel est pulvérisé le liquide. Cet élément est avantageusement une cellule dite à coefficient de température positif à variation brusque de la résistance pour limiter la puissance dissipée lorsque la température croît.

Selon une autre caractéristique, la température du gaz, après son passage à travers la paroi poreuse est mesurée à l'aide d'un élément à coefficient de température négatif avec un coefficient de variation sensiblement constant, la variation de résistance de cet élément étant utilisée pour une régulation en boucle fermée de l'apport thermique amené à la paroi poreuse. Lesdits éléments à coefficients de température négatifs (CTN) sont particulièrement bien adaptés à cet usage car ils présentent une très faible masse et donc une très faible inertie thermique avec un très fort coefficient de variation de la résistance de l'ordre de - 15 % par degré centigrade.

Selon un mode de réalisation préférentiel et pour éviter les transvasements du liquide anesthésiant, le réservoir à liquide est constitué par la bouteille de conditionnement commercial dont le bouchon est perforé par deux aiguilles assurant l'une l'introduction d'un gaz sous presion, l'autre le départ du liquide avec une pièce élastique assurant l'étanchéité autour du goulot.

L'invention sera décrite plus en détail ci-après sous forme d'un exemple de réalisation avec référence au dessin schématique ci-annexé dans lequel :

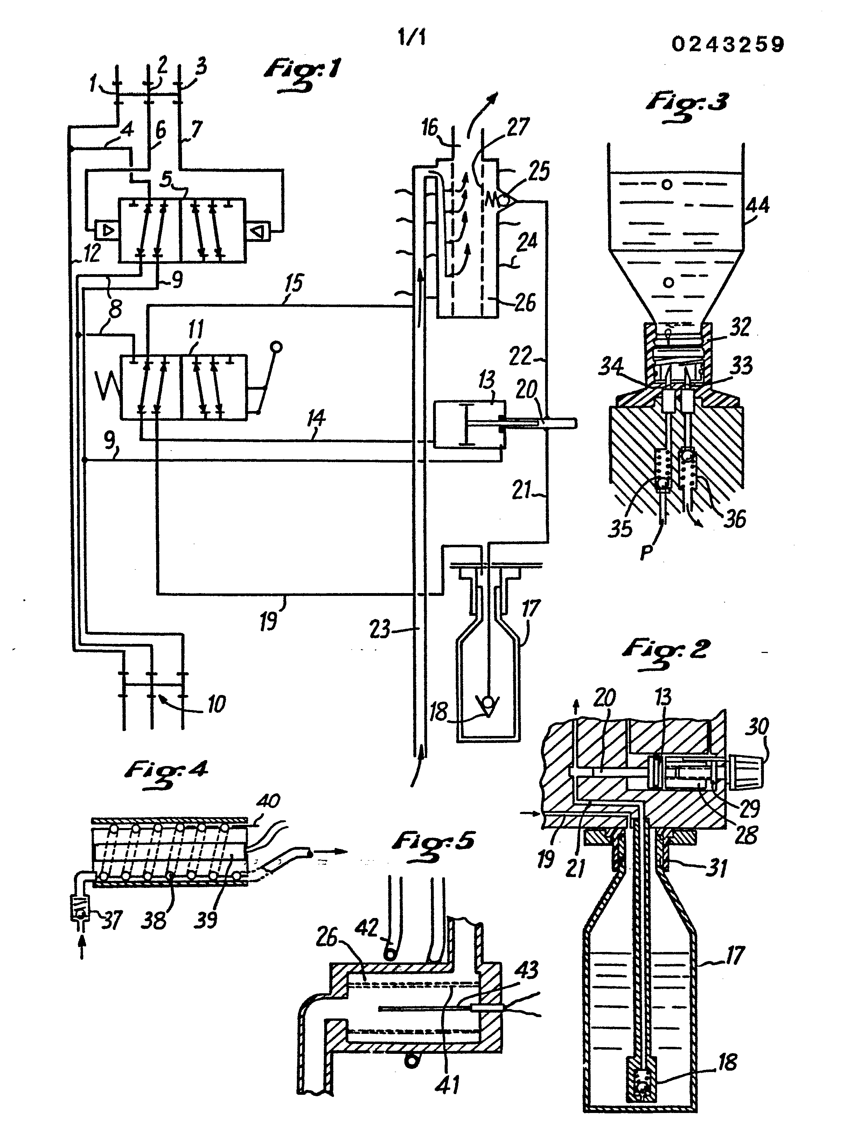

Figure 1 est un schéma d'ensemble de la partie doseur-évaporateur  conforme à l'invention d'un appareil respiratoire ; Figure 2 est une vue en coupe de détail du branchement de la bouteille contenant l'anesthésique ; Figure 3 est une vue de détail d'une variante de réalisation du branchement de la bouteille ; Figure 4 est une vue en coupe d'un évaporateur rentrant dans le dispositif et Figure 5 est une vue en coupe d'une variante de réalisation de la chambre d'évaporation.

L'appareil est raccordé au mélangeur de gaz par trois tuyaux d'arrivée 1, 2 et 3, l'arrivée 1 étant reliée à l'alimentation en gaz sous la pression d'alimentation disponible aux robinets d'arrivée dans les salles d'opération (350 kPa) et les entrées 2 et 3 aux sources des impulsions caractéristiques du mélangeur ou de l'équipement de mesure qui produisent des impulsions de pression gazeuse. Ces impulsions groupées deux par deux définissent un volume caractéristique par exemple 1dm3 de mélange gazeux distribué. De l'entrée 1 le gaz à 350 kPa est envoyé, par une conduite

0243259

4, à une vanne à cinq voies et deux positions 5 tandis que les autres entrées 2 et 3 sont connectées respectivement, par les conduites 6 et 7, aux cylindres de commande de changement d'état de la vanne 5. Ladite vanne est d'un modèle dont le basculement ne demande qu'un effort faible tels que ceux dits "Métal-Métal sans joint". De cette vanne, par les tuyauteries 8 et 9, les deux impulsions ainsi régénérées, ainsi que la pression provenant de l'entrée 1 par la conduite 12 sont dirigées dans tous les cas de fonctionnement vers une prise 10 analogue à la prise d'entrée et destinée à alimenter d'autres appareils de dosage par exemple un humidificateur. La tuyauterie 8 dirige d'autre part une des impulsions vers une seconde vanne du type cinq-deux 11 à commande manuelle qui, dans une première position, envoie, au vérin 13 de commande de la pompe doseuse du liquide par la tuyauterie 14, un gaz sous faible pression côté fond tandis que l'impulsion venant de 8 est bloquée. Ce gaz à faible pression, prélevé sur le gaz alimentant l'appareil respiratoire est amené à la vanne 11 par la conduite 15. Dans une deuxième position de la vanne 11, la conduite 8 est mise en communication avec la conduite 14 et alimente ainsi le côté fond du vérin 13, le côté tige dudit vérin étant alimenté directement par la conduite 9. On voit alors que, dans la première position de la vanne 11, le vérin reste immobilisé puisque sa chambre du côté fond est alimentée avec une pression insuffisante tandis que, dans la seconde position, le vérin fait un mouvement aller-retour en étant alimenté normalement alternativement sur ses deux faces. Par ailleurs, dans la seconde position de la vanne, la tuyauterie 15 est raccordée à la conduite 19 qui assure une surpression sur le liquide anesthésique comme expli-

qué ci-après.

Le circuit pour le liquide anesthésique est le suivant. Ce liquide est contenu dans une bouteille 17 qui est le conditionnement du commerce et qui est maintenue par enfoncement de son goulot dans une bague élastique qui assure son maintien et l'étanchéité. Un clapet 18 plonge dans le liquide. La conduite 19 évoquée plus haut amène la pression du gaz destiné au malade de façon à obtenir une légère surpression au-dessus du liquide pour empêcher son évaporation et aider le remplissage du circuit en particulier au moment de la purge. La pompe de dosage 20 est placée au-dessus du clapet 18 et y est reliée par la conduite 21. Elle est actionnée par le vérin 13 et elle refoule le liquide par la conduite 22 jusqu'au clapet de sortie 25 taré pour s'ouvrir vers 300 à 400 kPa. Le liquide anesthésique sortant par le clapet 25 est introduit dans la zone d'évaporation.

Le circuit du gaz respiratoire envoyé au malade est le suivant. Ce gaz arrive par la conduite 23 venant du mélangeur et passe aussitôt dans la zone annulaire périphérique de la chambre 26 où il évapore le liquide pulvérisé depuis le clapet 25 en empruntant l'apport thermique nécessaire à l'ambiance extérieure grâce aux ailettes d'échanges 24 dont est munie la paroi de la chambre 26, puis il traverse la paroi poreuse tubulaire 27 pour être envoyé vers l'utilisation par la conduite 16.

La figure 2 représente le détail de l'ensemble constitué par la bouteille 17, la pompe 20 et de son vérin 13 de commande. La course dudit vérin 13, et donc celle de la pompe, est régulée par une butée arrière 28 commandée par un ensemble vis-écrou 29. Cet ensemble vis écrou est lui-même commandé par un bouton à affichage 30. Les chiffres apparaissant sur ce bouton à affichage sont donc significatifs de la course sur la pompe 20. Celle-ci fonctionnant à raison d'un coup pour une quantité de gaz connue, généralement 1dm3, l'affichage indique en définitive le dosage du produit anesthésique ou de la vapeur d'eau. On voit également sur ce schéma la bague élastique 31 de maintien de la bouteille qui en assure aussi l'étanchéité.

La plupart des produits anesthésiques étant des hydrocarbures légers halogénés, donc des solvants extrèmement puissants, le dispositif décrit ci-dessus nécessite des joints résistant à ces solvants ou, tout au moins, ne présentant pas de gonflement trop important gênant le fonctionnement. Ces qualités sont disponibles aujourd'hui aisément, par exemple le polytétrafluoréthylène (PTFE) sous forme de joint à lèvres minces avec ressort de compression, ou le caoutchouc fluocarboné en bagues "O ring", ou encore les polyuréthanes moulés, la peau de moulage étant une très bonne barrière. Ceci est valable surtout pour les parties en contact avec le liquide, les vapeurs étant beaucoup moins agressives.

Un autre montage de la bouteille de liquide anesthésique est représenté sur la figure 3. La bouteille 44 y est utilisée munie de son bouchon, retournée tête en bas et maintenue dans une bague élastique 32. Dans cette position, le bouchon est perforé par deux aiguilles creuses 33, 34 qui s'étanchent dans la zone intérieur du bouchon. L'une des deux aiguilles 34 amène la surpression P à travers un clapet 35, destiné à interdire tout passage du liquide par ce circuit, et l'autre alimentant, à travers le clapet de pompe 36, la pompe proprement dite. Dans ce circuit, la visualistion du débit effectif sera le dégagement des bulles de pressurisation dans la bouteille.

Il est possible que les liquides que l'on ait à évaporer ne soient pas assez volatils pour se suffire de l'apport thermique asuré par les ailettes 24 de l'évaporateur. Il est également possible que l'on veuille réguler la température du gaz destiné au patient.

Dans ce cas, il est nécessaire de fournir une énergie thermique, tant pour l'évaporation que pour la régulation. Pour l'évaporation, la Figure 4 représente une solution simple. Le liquide L sortant de la pompe par le clapet 37 transite par une zone tubulaire 38 maintenue à une température légèrement supérieure à sa température d'ébullition par une résistance électrique 39 et une détection de température 40. Toutefois, dans la très grande généralité des cas, l'apport thermique des vapeurs ainsi générées, assuré par cet évaporateur, est insuffisant pour amener le gaz à la température voulue (généralement 37°C). La présente invention propose d'utiliser en échangeur thermique la paroi poreuse 27 prévue pour empêcher le passage des gouttelettes, laquelle est réalisée en métal fritté et présente de ce fait, une surface d'échange considérable : celle des grains de métal. Il suffit de la chauffer dans toute sa masse. La figure 5 représente une telle solution. La barrière poreuse 41 est réalisée en un matériau magnétique par exemple du nickel fritté et est combinée avec un générateur de chauffage 42 constitué par une spire de transformateur à très basse tension alimentée en 50 Hz et utilisant la masse de la paroi poreuse comme noyau secondaire. Une sonde de température 43 à résistance assure la régulation de température du gaz sortant en agissant sur le primaire dudit transformateur.

On peut aussi utiliser une paroi poreuse amagnétique chauffée par induction à haute fréquence par une bobine intérieure placée dans la même position que la résistance 39, ce positionnement à l'intérieur étant destiné à limiter les pertes thermiques.

Certains composants sont particulièrement adaptés à l'utilisation en chauffage par effet Joule, par exemple les modules à coefficient de température positif au titanate de baryum et de strontium dont la résistance varie brusquement à une certaine température. On connaît ainsi une cellule revêtue de résine aux époxydes dont la résistance de 8 Ohms à 25°C passe brusquement à 40 Ohms à 125°C laquelle constitue un moyen très simple et très sûr de limitation de la puissance. D'autres éléments de la même technologie à coefficient de température négatif ou positif de très petites dimensions et de résistance importante ont des variations de résistance de l'ordre de 10 à 15 % par degré centigrade et constituent ainsi un capteur de température très efficace, très sensible et très bon marché, tant pour la surveillance d'une élévation anormale de température dans la zone de vaporisation que pour la régulation de température du gaz destiné au malade.

Revendications

1. Un doseur-évaporateur de liquide dans un circuit de gaz qui combine un dispositif de mesure du débit gazeux, une pompe doseuse de volumes du liquide et une chambre de vaporisation du liquide débité dans le gaz,
caractérisé en ce que la pompe doseuse (13-20) fonctionne selon un cycle asservi à des impulsions émises par le dispositif de mesure du débit gazeux, chaque impulsion correspondant à un volume gazeux débité fixe et constant, la pompe doseuse (13-20) de course réglable (28-30) effectuant un cycle par impulsion reçue du dispositif de mesure et la chambre de vaporisation étant constituée par une enceinte (26) traversée par le courant gazeux dans laquelle les volumes de liquide dosés sont pulvérisés (25), ladite enceinte étant séparée de la sortie des gaz par une paroi poreuse (27).

2. Un doseur évaporateur selon la revendication 1,
caractérisé en ce que la paroi poreuse (27) est tubulaire.

3. Un doseur-évaporateur selon l'une quelconque des revendications 1 et 2,
caractérisé en ce que la paroi poreuse (27) présente une capacité et une conductibilité thermiques importantes pour réguler et uniformiser la température des gaz en sortie.

4. Un doseur évaporateur selon l'une quelconque des revendications 1 à 3,

caractérisé en ce que la paroi poreuse (41) est réalisée en poudre magnétique frittée et est entourée par un enroulement (42) électrique parcouru par un courant alternatif basse tension qui est régulé pour assurer la température en sortie voulue du mélange gazeux anesthésique.

5. Un doseur-évaporateur selon l'une quelconque des revendications 1 à 3, caractérisé en ce que la paroi poreuse (27) est réalisée en un matériau amagnétique et est chauffée par une bobine d'induction haute fréquence (39) placée à l'intérieur de ladite paroi poreuse tubulaire.

6. Un doseur-évaporteur selon l'une quelconque des revendications 1 à 5, caractérisé en ce que l'évaporation est assurée, avant le passage à travers la paroi poreuse (27), par un élément formant résistance électrique sur lequel est pulvérisé le liquide.

7. Un doseur-évaporteur selon l'une quelconque des revendications 1 à 6, caractérisé en ce que la température du gaz, après son passage à travers la paroi poreuse (27) est mesurée à l'aide d'un élément à coefficient de température négatif avec un coefficient de variation sensiblement constant, la variation de résistance de cet élément étant utilisée pour une régulation en boucle fermée de l'apport thermique amené à la paroi poreuse.

8. Un doseur évaporteur selon l'une quelconque des revendications 1 à 7,

caractérisé en ce que le réservoir à liquide est constitué par la bouteille (44) de conditionnement commercial dont le bouchon est perforé par deux aiguilles (33-34) assurant l'une (33) l'introduction d'un gaz sous presion, l'autre (34) le départ du liquide, avec une pièce élastique (32) assurant l'étanchéité autour du goulot.

0243259

*Fig.1*

*Fig.3*

*Fig.4*

*Fig.5*

*Fig.2*